# EUROPEAN PATENT SPECIFICATION

(11) **EP 3 846 701 B1**
(45) Date of publication and mention of the grant of the patent: **20.07.2022**
(21) Application number: 19763110.4
(22) Date of filing: 29.08.2019
(51) Int. Cl.: A61B 17/15

(54) **TIBIAL RESECTION GUIDE DEVICE**
TIBIARESEKTIONSFÜHRUNG
DISPOSITIF DE GUIDAGE DE RÉSECTION TIBIALE

(30) Priority: 04.09.2018 IT 201800008346
(43) Date of publication of application: 14.07.2021
(73) Proprietor: Medacta International SA, 6874 Castel San Pietro (CH)
(72) Inventor: SICCARDI, Francesco, 6874 Castel San Pietro (CH); BERNARDONI, Massimiliano, 6874 Castel San Pietro (CH); BECCARI, Alessio, 6874 Castel San Pietro (CH); BURGASSI, Fabio, 6874 Castel San Pietro (CH); HOWELL, Stephen, 6874 Castel San Pietro (CH)
(74) Representative: Porta & Consulenti Associati S.p.A.
(86) International application number: PCT/IB2019/057272
(87) International publication number: WO 2020/049421

(56) References cited:
- WO-A1-03/013371
- WO-A1-2016/205454
- US-A- 6 077 270
- US-A1- 2014 228 851

## Description

The present invention relates to a tibial resection guide device, in particular for tibial resection in a surgical operation for the reconstruction of the knee joint.

Document US6077270 A discloses an exemplary tibial resection guide device.

In an operation for the reconstruction of the knee joint it is often necessary to implant knee prostheses for restoring the functionality of the joint.

Such knee prostheses typically comprise a femoral prosthetic component and a tibial prosthetic component, designed for the fixing to the femur and to the tibia respectively, and coupling surfaces designed to house an articulated movement of the knee. The implantation of the femoral and tibial prosthetic component requires the resection of the femur and of the tibia, respectively, in order to realise surfaces able to house and retain the prostheses to be implanted.

The complex geometry of the knee joint, together with the complex distribution of mechanical loads thereon, make the correct alignment of the prosthetic components and the safe positioning on the tibia and on the femur of the patient extremely important.

For this reason, guide devices have been developed to perform a tibial resection that enable the surgeon to create a cutting plane that enables the recovery of the alignment of the patient's lower limb.

Known guide devices enable the tibial resection to be performed along a cutting plane that can be oriented with respect to the mechanical axis of the tibia, i.e. with respect to the axis that passes through the centre of the knee joint and through the centre of the ankle joint.

In particular, the surgeon performs an alignment of a guide rod with the mechanical axis of the tibia and subsequently orients the cutting plane choosing a distance, along the proximal-distal axis, of the cutting plane from a reference such as the medial condyle or the lateral condyle of the tibia, inclining the cutting plane with respect to a medial-lateral axis (varus/valgus angle) and inclining the cutting plane with respect to an anteroposterior axis (slope angle).

Medical studies have highlighted that a certain proportion of the population has an alignment of the lower limb that diverges from the neutral alignment, i.e. it has a native joint line having a certain degree of constitutional varus or valgus.

In such subjects, who from when they stop growing have a configuration that diverges from the neutral alignment, it could be unnatural and harmful to restore a neutral alignment after the implantation of a tibial prosthesis and it may be more successful to restore a kinematic alignment of the tibia, preserving the patient's native joint line.

However, the Applicant has noted that known guide devices, being configured for alignment with the mechanical axis of the tibia, do not enable precise and reliable use when the surgeon decides to use an operating technique of kinematic alignment of the tibia. In fact, the Applicant has noted that guide devices of the prior art force surgeons who decide to preserve the patient's native joint line to orient the cutting plane with respect to a medial-lateral axis and with respect to an anteroposterior axis in a non-assisted and uncontrolled way, i.e. performing an alignment by visual comparison between the cutting plane and the patient's native joint line.

The Applicant has found that such an unassisted and uncontrolled orientation of the cutting plane could require, once the tibial resection is complete, subsequent corrections to obtain the desired alignment with consequent bone sacrifice and extension of the operation.

The Applicant has perceived that it would be useful to be able to have available a guide device for tibial resection that enables assisted and controlled orientation of the cutting plane with respect to the patient's native joint line at least along a medial-lateral axis and an anteroposterior axis.

The Applicant has perceived that, by taking as a direct reference for the definition of the patient's native joint line at least two distinct points on the tibial plateau, it is possible to use such two distinct points as a reference for the orientation of the cutting plane.

The Applicant has also perceived that by choosing one of such two distinct points on the medial condyle and the other on the lateral condyle, it is possible to obtain a precise reference of the inclination of the cutting plane with respect to a medial-lateral axis.

The Applicant has also perceived that by choosing both of such two distinct points on the medial condyle or on the lateral condyle, it is possible to obtain a precise reference of the inclination of the cutting plane with respect to an anteroposterior axis.

Therefore, the present invention relates, according to a first aspect, to a guide device for tibial resection comprising:
a guide jig comprising a slot provided for accepting and guiding the blade of a surgical saw;
a first feeler provided for coming into contact with a point or an area of a tibial plateau;
a second feeler provided for coming into contact with a point or an area of a tibial plateau;
wherein the first feeler and the second feeler can be rotated with respect to one another and to the guide jig around the same rotation axis, wherein the first feeler can be translated with respect to the second feeler and to the guide jig along a first sliding direction contained within a first sliding plane, and wherein the second feeler can be translated with respect to the first feeler and to the guide jig along a second sliding direction contained within a second sliding plane parallel to the first sliding plane.

The possibility of the first and the second feeler to rotate with respect to one another and to the guide jig and the possibility of the first and the second feeler to translate, respectively, along the first and the second sliding direction allow each feeler to reach any area of the tibial plateau.

This allows the feelers to be used as physical and direct references on the tibial plateau for aligning the guide jig with the patient's main varus/valgus angle and/or for aligning the guide jig with the patient's main slope angle. The cutting jig can therefore be aligned in an assisted and controlled way with the patient's joint line allowing the surgeon to perform objective kinematic alignment of the guide jig.

In the same way, the first and the second feeler can be used to determine the cutting height at which to position the cutting jig, enabling the simultaneous and objective determination of the varus/valgus angle and the cutting height or the slope angle and the cutting height.

In particular, placing in contact the first feeler with a preselected point on the medial condyle and the second feeler with a pre-selected point on the lateral condyle of the tibial plateau, the guide jig (and with it the slot for the surgical saw) can be aligned with an imaginary straight line passing through the two pre-selected points. By choosing such two points in such a way that the imaginary straight line passing through them is parallel to the patient's native joint line, the guide jig can be aligned (by inclining it with respect to a medial-lateral axis) with the varus/valgus angle of the patient's joint line and, at the same time, it can be placed at the optimal cutting height (along a proximal-distal axis).

By placing in contact both the first feeler and the second feeler with pre-selected points of the medial condyle or the lateral condyle of the tibial plateau in respective anterior or posterior positions, the guide jig (and with it the slot for the surgical saw) can be aligned (inclining it with respect to an anterior-posterior axis) with an imaginary straight line passing through such two points. By choosing the two points in such a way that the imaginary straight line passing through them is parallel to the patient's native slope line, the guide jig can be aligned with the patient's native slope angle and, at the same time, it can be placed at the pre-selected cutting height (along a proximal-distal axis).

Also described herein although not falling within the scope of the claims is a tibial resection method comprising:
providing a guide jig equipped with a first and a second feeler;
adjusting the varus/valgus angle and the cutting height of the guide jig by placing in contact with the first and the second feeler respectively a pre-selected point of the medial condyle and of the lateral condyle of the tibia;
anchoring the guide jig to the tibial bone;
inserting a surgical saw into the guide jig and cutting the tibial bone.

Also described herein although not falling within the scope of the claims is a tibial resection method
comprising:
providing a guide jig equipped with a first and a second feeler;
adjusting the slope angle and the cutting height of the guide jig by placing in contact with the first and the second feeler two pre-selected points of the medial condyle or of the lateral condyle of the tibia spaced out along an anteroposterior axis;
anchoring the guide jig to the tibial bone;
inserting a surgical saw into the guide jig and cutting the tibial bone.

The guide jig according to the second and third aspect may be the guide jig or the guide device according to the first aspect of the invention.

The expression "assisted and controlled" alignment or positioning means unambiguous alignment or positioning that is not subject to visual comparison between imaginary lines and physical objects, in particular it means alignment or positioning determined by contact between physical elements (e.g. a feeler and a portion of bone).

In the present description and subsequent claims a standard anatomic reference system is also used to which the present invention may refer.

In particular, a vertically direct and parallel axis to the gravitational force vector is indicated as the proximal-distal axis; a perpendicular axis to a proximal-distal axis and crossing the patient's body from left to right is indicated as a medial-lateral axis; a perpendicular axis both to a medial-distal axis and to a proximal-distal axis is indicated as an anteroposterior axis.

The plane of symmetry of the patient's body containing both a proximal-distal axis and an anteroposterior axis (which therefore divides the patient's body into a right portion and a left portion) is indicated as a sagittal plane; the vertical plane that contains a proximal-distal axis and a medial-lateral axis and that passes through the centre of mass of the patient's body (which therefore divides the patient's body into an anterior portion and a posterior portion) is indicated as a frontal or coronal plane; the perpendicular plane both to the sagittal plane and to the frontal plane passing through the centre of mass of the patient's body (which therefore divides the patient's body into an upper portion and a lower portion) is indicated as a horizontal or transverse plane.

The term "anterior" means directed towards the front part of the patient's body along an anteroposterior axis; the term "rear" means directed towards the rear part of the patient's body along an anteroposterior axis; the term "upper" means directed towards an upper part (head) of the patient's body along a proximal-distal axis; the term "lower" means directed towards the lower part (feet) of the patient's body along a proximal-distal axis; the term "medial" means directed towards the sagittal plane along a medial-lateral axis; the term "lateral" means directed away from the sagittal plane along a medial-lateral axis.

An anteroposterior direction is a direction directed from the front part to the rear part (or vice versa) of the patient's body. A proximal-distal direction is a direction directed from the lower part to the rear part (or vice versa) of the patient's body. A medial-lateral direction is a direction directed from the left part to the right part (or vice versa) of the patient's body.

When in reference to a limb or a bone, the term "proximal" means an end closest to the patient's heart and the term "distal" means an end furthest from the patient's heart.

The term "varus", in orthopaedic language, means an internal angle (towards the sagittal plane) of the distal part of a bone or of a limb; the term "valgus", in orthopaedic language, means an outer angle (away from the sagittal plane) of the distal part of a bone or of a limb. The term "varus/valgus angle" means the angle formed between a medial-lateral axis passing through the frontal plane and the outline on the frontal plane of a reference plane perpendicular to the frontal plane passing through a pre-selected point of the medial condyle and a pre-selected point of the lateral condyle.

The term "slope angle" means the angle formed between an anteroposterior axis passing through the sagittal plane and the outline on the sagittal plane of a reference plane perpendicular to the sagittal plane and passing through two pre-selected points of the medial condyle or of the lateral condyle spaced out from each other in the anteroposterior direction.

The present invention may comprise, according to any one of three aspects, one or more of the following preferred characteristics, taken individually or in combination.

Preferably, there are two degrees of freedom of each feeler with respect to the guide jig; a translation along the respective sliding direction and a rotation about the rotation axis.

Preferably, the rotation axis, the first sliding plane and the second sliding plane have respective pre-set inclinations relative to the slot of the guide jig.

In other words, the rotation axis and the two sliding planes are preferably fixed with respect to the guide jig.

Preferably, the first and the second sliding plane are perpendicular to said rotation axis.

In this way, the height measured along the rotation axis of the first and of the second feeler does not change during the translation along the first and the second sliding direction of the first and of the second feeler.

The rotation axis is preferably perpendicular to the slot of the guide jig and the first and the second sliding plane are preferably parallel to the slot of the guide jig.

In this way, the first and the second feeler directly define the slope angle and/or the varus/valgus angle that the slot of the guide jig must assume after performing further calculations that consider an initial inclination of the slot of the guide jig with respect to the rotation axis.

Alternatively, the rotation axis can be inclined with respect to the slot of the guide jig so as to provide the guide jig with a pre-set varus/valgus angle or with a pre-set slope angle.

In particular, the rotation axis can form a different pre-defined angle from 90° with an anteroposterior axis contained in a plane defined by the slot of the guide jig. In this case, the guide jig has a pre-set slope angle that has a substantially equal value to the difference between 90° and the aforementioned predefined angle. The rotation axis can form, additionally or alternatively, a different pre-defined angle from 90° with a medial-lateral axis contained in a plane defined by the slot of the guide jig. In this case, the guide jig has a pre-set varus/valgus angle that has a substantially equal value to the difference between 90° and the aforementioned predefined angle.

Preferably, the first feeler comprises a first end portion that develops along a first contact direction, inclined with respect to the first sliding plane, the first end portion being configured to come into contact with a point or an area of a tibial plateau, the projection of the first contact direction on the first sliding plane being inclined with respect to the first sliding direction. Preferably, the second feeler comprises a first end portion that develops along a second contact direction, inclined with respect to the second sliding plane, the first end portion being configured to come into contact with a point or an area of a tibial plateau, the projection of the second contact direction on the second sliding plane being inclined with respect to the second sliding direction. In other words, the first end portion of the first feeler and/or the second feeler can be inclined in the medial or lateral direction (i.e. towards the sagittal plane or away from the sagittal plane).

In this way, the first feeler and/or the second feeler can reach areas of the tibial plateau that would be precluded or however difficult to reach such as front or rear areas of the tibial plateau very proximal to the tibial crest.

Preferably the first feeler is spaced apart by a pre-set distance along the rotation axis from the second feeler, the first end portion of the first feeler having an extension, in a direction parallel to the rotation axis, that is equivalent to the extension, in a direction parallel to the rotation axis, of the first end portion of the second feeler reduced by a pre-set amount.

The second feeler can therefore be superposed with the first feeler so as to make them rotatable within a common rotation axis. The superposition of the two feelers places them on planes that are parallel to each other but distanced in a proximal-distal direction. By making the first end portion of the second feeler shorter than the first end portion of the first feeler (by a predetermined amount), the two first end portions can come into contact with different areas of the tibial plateau, creating a precise kinematic alignment of the guide jig.

In the case in which the patient's tibial plateau is not subject to cartilaginoid and/or bone wear, the mentioned predetermined amount can be equal to the distance in the proximal-distal direction that separates the first feeler from the second feeler. In this way, the first end portions of the first and the second feeler lie on a plane containing a medial-lateral axis and an anteroposterior axis, i.e. they lie at the same height.

In the case in which the patient's tibial plateau is subject to cartilaginoid and/or bone wear, the mentioned predetermined amount can be equal to the distance in the proximal-distal direction that separates the first feeler from the second feeler minus an amount equal to the height of the missing cartilage and/or bone. In this way, the different height at which the first end portions of the feelers lie compensates for the cartilaginoid and/or bone wear of the tibial plateau.

Preferably, a support body for the first and the second feeler can be inserted into the guide jig, the support body comprising tightening members that can be switched from a free positioning condition, in which the first and the second feeler can be rotated and translated with respect to the support body, and a locking condition, in which the first and the second feeler are held in position with respect to the support body.

In this way, the first and the second feeler can rotate independently about the rotation axis and with respect to the support jig and translate independently along the respective first and second sliding direction, allowing a precise positioning of the two feelers on the tibial plateau, and can be locked in the position reached in order to act as a reference for the orientation of the guide jig.

Preferably, a single support body is provided, active on both the first and the second feeler.

Preferably, said guide jig comprises a plurality of constraining members spaced apart from one another to accept and hold the support body in different positions.

The support body can therefore be associated with predetermined different areas of the guide jig as a function of the main anatomy of the patient and the specific tibial resection to be performed. Preferably, an extramedullary or intramedullary alignment guide is provided; said cutting jig being associated with said alignment guide so that it can be moved by said alignment guide.

This allows the guide jig to be positioned with respect to the patient's tibia and to support it during the kinematic alignment operations of the guide jig.

Preferably, the alignment guide comprises adjustment members to translate the guide jig along a direction parallel to said rotation axis and to rotate the guide jig around two axes that are perpendicular to one another and perpendicular to the rotation axis.

The guide jig is preferably integral with a proximal end portion of the alignment guide, so as to move together with the alignment guide.

The adjustment members preferably act directly on the alignment guide, so that by acting on the alignment guide the guide jig can be oriented so as to align it with the joint line of the patient's tibia. In the event that an extramedullary alignment guide can be used, the adjustment members envisage the possibility to translate a distal end portion of the alignment guide along three mutually perpendicular adjustment directions. Such three mutually perpendicular directions are preferably parallel or aligned with an anteroposterior direction, a proximal-distal direction and a medial-lateral direction, respectively.

Preferably, a first adjustment direction is substantially directed in the proximal-distal direction and the actuation of the adjustment members in such first adjustment direction causes a translation in the proximal-distal direction of the guide jig that allows the cutting height at which to position the guide jig to be selected. Preferably, a second adjustment direction is substantially directed in the anteroposterior direction and the actuation of the adjustment members in such second adjustment direction causes a rotation about a substantially medial-lateral axis of the guide jig that allows the slope angle at which to position the guide jig to be selected.

Preferably, a third adjustment direction is substantially directed in the medial-lateral direction and the actuation of the adjustment members in such third adjustment direction causes a rotation about a substantially anteroposterior axis of the guide jig that allows the varus/valgus angle at which to position the guide jig to be selected.

The adjustment members are preferably actuated so that the first and the second feeler come into contact with the pre-selected points of the tibial plateau for the kinematic alignment of the cutting jig.

In the event in which the surgeon decides to perform an assisted and controlled alignment with the varus/valgus angle, the slope angle is preferably adjusted first.

In this case, the cutting jig is preferably rotated first about a substantially medial-lateral axis so that the slot of the cutting jig is parallel, in the sagittal view, to the medial or lateral condyle. The two feelers are preferably rotated and translated so that they are positioned respectively at two pre-selected points on the medial and lateral condyle.

The two feelers are brought into contact with the two pre-selected points by rotating the cutting jig about a substantially anteroposterior axis and translating the cutting jig in the proximal-distal direction, so as to set the varus/valgus angle and the cutting height.

In the event in which the surgeon decides to perform an assisted and controlled alignment with the slope angle, the varus/valgus angle is preferably adjusted first.

In this case, the cutting jig is preferably rotated first about a substantially anteroposterior axis so that the slot of the cutting jig is aligned, in the front view, with the patient's joint line.

The two feelers are preferably rotated and translated so that they are positioned respectively at two pre-selected points on the medial or lateral condyle.

At this point, the two feelers are brought into contact with the two pre-selected points by rotating the cutting jig about a substantially medial-lateral axis and translating the cutting jig in the proximal-distal direction, so as to set the slope angle and the cutting height. Further features and advantages of the invention will be more evident from the following description of preferred embodiments thereof made with reference to the appended drawings. In such drawings:
- figure 1 is a perspective view of axes and planes of reference useful for better illustrating the present invention;
- figure 2 is a perspective view of some components of a guide device for tibial resection according to the present invention;
- figure 3 is a sagittal view of the components of figure 2;
- figure 4 is a perspective view of the guide device for tibial resection of figure 2 applied to a proximal portion of a tibia;
- figure 5 is a perspective view of the guide device for tibial resection of figure 4 in a different operating configuration;
- figure 6 is a view on a horizontal plane of the guide device for tibial resection of figure 4;
- figure 7 is a perspective view of the guide device for tibial resection of figure 5; and
- figure 8 is a perspective view of some components of the guide device for tibial resection of figures 4 and 5.

A guide device for tibial resection according to the present invention is indicated overall by number 10.

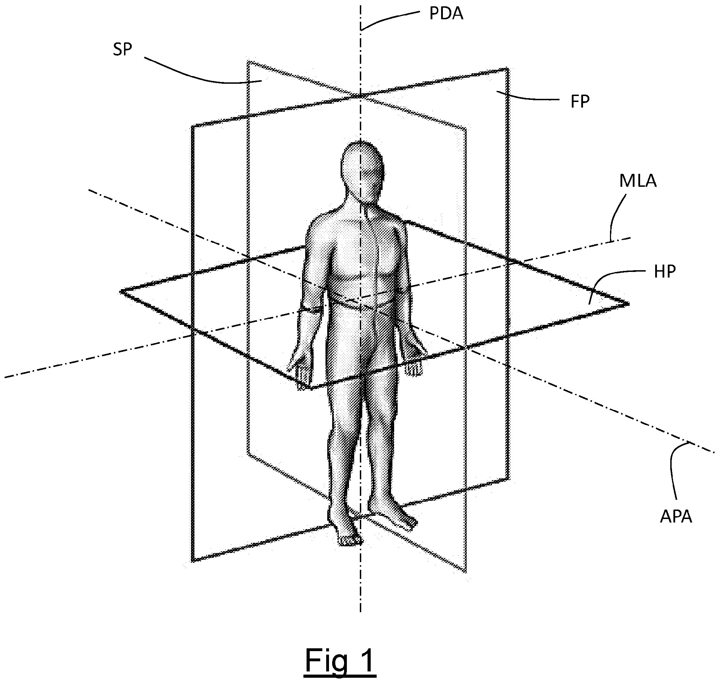

Figure 1 indicates a sagittal plane SP, a frontal or coronal plane FP and a horizontal or transverse plane HP with reference to the patient's body.

The sagittal plane SP is substantially vertical and ideally divides the human body into two symmetrical half parts. The frontal plane FP is substantially vertical and ideally divides the human body into a front part and a rear part. The horizontal plane FP is substantially horizontal and ideally divides the human body into an upper part and a lower part. As illustrated in figure 1, the sagittal plane SP, the frontal plane FP and the horizontal plane HP are perpendicular to each other.

Figure 1 also indicates a proximal-distal axis PDA, a medial-lateral axis MLA and an anteroposterior axis APA perpendicular to one another. The proximal-distal axis PDA is contained in the sagittal plane SP and in the frontal plane FP, the medial-lateral axis MLA is contained in the horizontal plane HP and in the frontal plane FP and the anteroposterior axis APA is contained in the horizontal plane and in the sagittal plane SP.

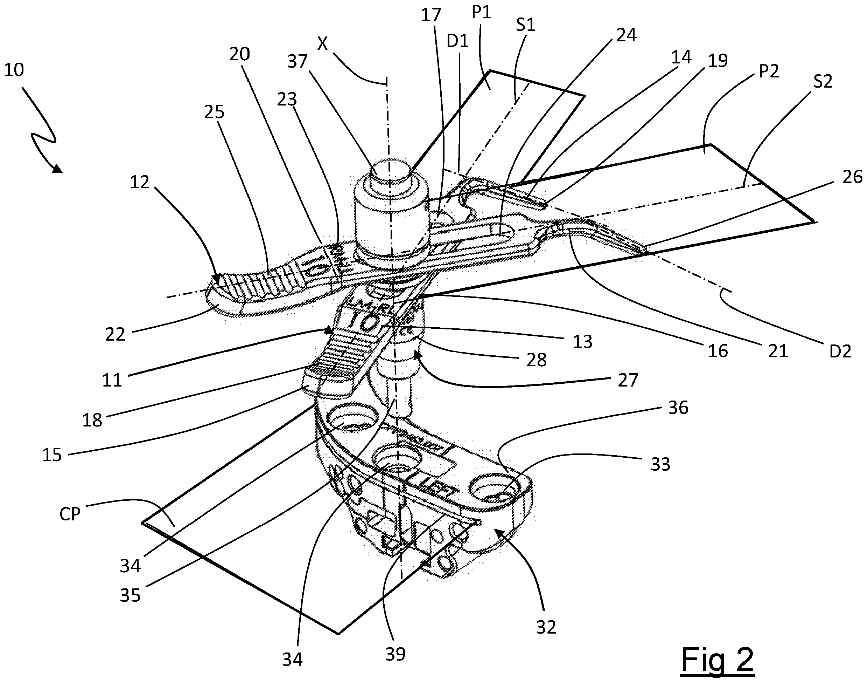

With particular reference to figure 2, the guide device 10 comprises a first 11 and a second feeler 12 provided to come into contact with points or areas of a tibial plateau 100.

The first feeler 11 comprises an elongated body 13 comprising a first end portion 14, a second end portion 15 opposite the first 14 and a central portion 16 that extends between the first 14 and the second 15 end portion.

The central portion 16 comprises a slot 17 for allowing the first feeler 11 to translate along a first sliding direction S1 so as vary the relative distance between the first end portion 14 and a fixed reference.

The first sliding direction S1 is contained in a first sliding plane P1, as illustrated in figure 2.

The second end portion 15 comprises a gripping area 18 predisposed to be gripped by a user (for example by the surgeon) in order to be able to move the first feeler 11 along the first sliding direction S1 and about a rotation axis X. The gripping area 18 is knurled (i.e. provided with a plurality of reliefs and hollows) in order to facilitate the grip thereof.

The first end portion 14 is tapered and ends with a rounded or acuminated contact area 19 provided to come into contact with a reduced area (tending to a point) of the tibial plateau 100.

The first end portion 14 extends along a first contact direction D1 which is not aligned with the first sliding direction S1 of the first feeler.

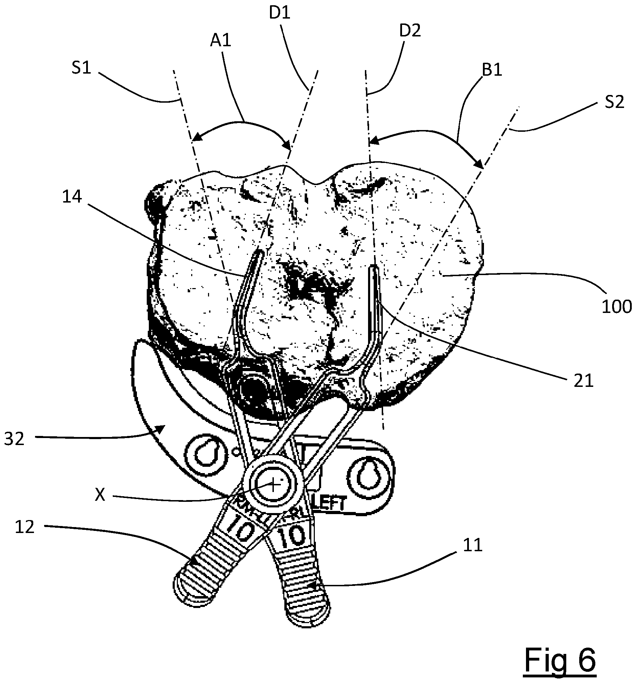

In particular, the first contact direction D1 is inclined in the medial or lateral direction with respect to the first sliding direction S1, so as to be diverted with respect to the first sliding direction S1 towards the sagittal plane SP or away from the sagittal plane SP, as is better illustrated in figure 6. In other words, the projection of the first contact direction D1 on the first sliding plane P1 forms a different angle A1 from zero with the first sliding direction S1. Such angle A1 can be comprised between about 5° and about 90°, preferably comprised between about 10° and about 60°, more preferably about 30°. In any case, the inclination in the medial or lateral direction and the angle A1 can be selected as a function of the main anatomy of the tibial plateau 100 and the position of the points on the tibial plateau that the surgeon decides to choose as a reference for the alignment of the guide jig.

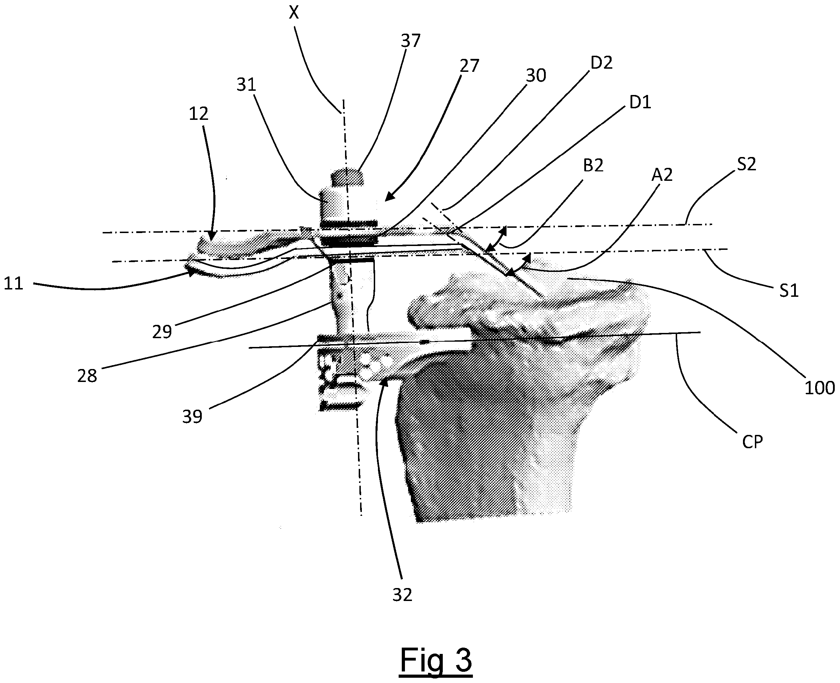

The first contact direction D1 is also inclined in the distal direction with respect to the first sliding direction S1, so as to be diverted with respect to the first sliding direction S1 away from the horizontal plane HP, as is illustrated better in figure 3. In other words, the projection of the first contact direction D1 on a perpendicular plane to the first sliding plane P1 and containing the first sliding direction S1 forms a different angle A2 from zero with the first sliding direction S1. Such angle A2 can be comprised between about 5° and about 90°, preferably comprised between about 10° and about 60°, more preferably about 45°. In any case, such angle A2 can be selected as a function of the main anatomy of the tibial plateau 100 and the position of the points on the tibial plateau that the surgeon decides to choose as a reference for the alignment of the guide jig.

The second feeler 12 comprises an elongated body 20 comprising a first end portion 21, a second end portion 22 opposite the first 21 and a central portion 23 that extends between the first 21 and the second 22 end portion.

The central portion 23 comprises a slot 24 for allowing the second feeler 12 to translate along a second sliding direction S2 so as vary the relative distance between the first end portion 21 and a fixed reference.

The second sliding direction S2 is contained in a second sliding plane P2, as illustrated in figure 2.

The second end portion 22 comprises a gripping area 25 provided to be gripped by the surgeon in order to be able to move the second feeler 12 along the second sliding direction S2 and about the rotation axis X. The gripping area 25 is knurled (i.e. provided with a plurality of reliefs and hollows) in order to facilitate the grip thereof.

The first end portion 21 is tapered and ends with a rounded or acuminated contact area 26 provided to come into contact with a reduced area (tending to a point) of the tibial plateau 100.

The first end portion 21 extends along a second contact direction D2 which is not aligned with the second sliding direction S2 of the second feeler 12.

In particular, the second contact direction D2 is inclined in the medial or lateral direction with respect to the second sliding direction S2, so as to be diverted with respect to the second sliding direction S2 towards the sagittal plane SP or away from the sagittal plane SP, as is better illustrated in figure 6. In other words, the projection of the second contact direction D2 on the second sliding plane P2 forms a different angle B1 from zero with the second sliding direction S2. Such angle B1 can be comprised between about 5° and about 90°, preferably comprised between about 10° and about 60°, more preferably about 30°. In any case, the inclination in the medial or lateral direction and the angle B1 can be selected as a function of the main anatomy of the tibial plateau 100 and the position of the points on the tibial plateau that the surgeon decides to choose as a reference for the alignment of the guide jig.

The second contact direction D2 is also inclined in the distal direction with respect to the second sliding direction S2, so as to be diverted with respect to the second sliding direction S2 away from the horizontal plane HP, as is illustrated better in figure 3. In other words, the projection of the second contact direction D2 on a perpendicular plane to the second sliding plane P2 and containing the second sliding direction S2 forms a different angle B2 from zero with the second sliding direction S2. Such angle B2 can be comprised between about 5° and about 90°, preferably comprised between about 10° and about 60°, more preferably about 45°. In any case, such angle B2 can be selected as a function of the main anatomy of the tibial plateau 100 and the position of the points on the tibial plateau that the surgeon decides to choose as a reference for the alignment of the guide jig.

For example, in the case of an assisted and controlled alignment of the guide device 10 with the patient's varus/valgus angle, the first contact direction D1 of the first end portion 14 of the first feeler 11 can be inclined in the medial direction and the second contact direction D2 of the first end portion 21 of the second feeler 12 can be selected inclined in the lateral direction (or vice versa), so that the two first end portions 14, 21 are substantially converging.

In the case of an assisted and controlled alignment of the guide device 10 with the patient's slope angle, the first contact direction D1 of the first end portion 14 of the first feeler 11 can be inclined in the same direction as the second contact direction D2 of the first end portion 21 of the second feeler 12.

The guide device 10 further comprises a support body 27 for the first 11 and the second feeler 12.

The support body 27 has a substantially cylindrical shape and enables the first 11 and the second feeler 12 to rotate about the common rotation axis X.

The rotation axis X is substantially aligned with a proximal-distal direction.

The first feeler 11 is inserted, through the slot 17, into a central portion 28 of the support body 27 which has dimensions such as to allow the slot to slide along the first sliding direction S1 with respect to the support body 27. The central portion 28 of the support body 27 is provided with a shoulder 29 on which the central portion 16 of the first feeler 11 rests (figure 3). The shoulder 29 defines an end stop at the degree of insertion of the first feeler 11 on the support body 27.

The second feeler 12 is also inserted, through the slot 24, into the central portion 28 of the support body 27 so as to be superposed with the first feeler 11. Between the first feeler 11 and the second feeler 12 an anti-friction washer 30 is positioned on which the second feeler 12 rests (figure 3). The anti-friction washer 30 defines an end stop at the degree of insertion of the second feeler 11 on the support body 27.

The support body 28 comprises tightening members 31 that can be switched from a free positioning condition to a locking condition. In the free positioning condition, the tightening members 31 enable free rotation about the rotation axis X of the first 11 and the second feeler 12 and free translation along the respective first S1 and second S2 sliding direction of the first 11 and second feeler 12. In the locking condition the tightening members 31 hold in position, by friction, the two feelers with respect to the support body 27. An example of tightening members 31 may be a presser element (for example a spring) coaxial to the support body 27 that by pressing the second feeler 12 on the first feeler 11 in the parallel direction to the rotation axis X locks by friction the two feelers in the position reached. A person skilled in the art is however aware that different types of tightening members 31 may be used for the purpose.

The guide device 10 comprises a guide jig 32 provided with constraining members 33 to accept and hold the support body 27 in a releasable way.

The constraining members 33 comprise a plurality of seats 34 predisposed to accept and retain an end portion 35 of the support body 27. The seats 34 are placed on an upper surface of the guide jig 32. The seats 34 are placed at a distance from one another so that the surgeon can choose the most suitable seat, and therefore the most suitable relative position between the feelers 11, 12 and the guide jig 32, as a function of the anatomy of the patient's tibial plateau and the points on the tibial plateau that the surgeon chooses to contact.

The end portion 35 of the support body 27 can be inserted to measure in any of the seats 34 so that the support body 27 is integral with the guide jig 32. In other words, when the support body 27 is inserted into the constraining members 33, the support body 27 cannot perform any translations with respect to the guide jig 32. Preferably, when the support body 27 is inserted into the constraining members 33, the support body 27 can rotate about the rotation axis X with respect to the guide jig 32 to facilitate the assembly operations of the support body on the guide jig 32. Each seat 34 comprises an abutment 36 for the end portion 35 of the support body 27 which defines an end stop for the insertion in a parallel direction to the rotation axis X of the support body 27 in the seat 34. The abutment 36 further defines the height of the two feelers 11, 12 with respect to the guide jig 32. It is to be noted that such height is constant and cannot be changed once the support body is inserted into the constraining members 33. Inside the end portion 35 of the support body 27 a peg may be provided (not illustrated) that is snap fitted into a housing with an arched extension (not illustrated) obtained in each seat 34, so as to prevent translations of the support body 27 with respect to the guide jig 32 along the rotation axis X. A person skilled in the art can however understand that other types of constraining mechanisms can be used for the purpose.

In any case, the support body 27 comprises a release mechanism 37 for removing the support body from the seat 34 in the guide jig 32. In the embodiment illustrated in the appended figures, the release mechanism 37 comprises a button 38 which acts on the constraining mechanism. By way of example, the button 38 can act on a spring (not illustrated) active on the peg of the end portion 35 of the support body for disengaging it from the housing in the seat 34.

The guide jig 32 further comprises a slot 39 predisposed to accept and guide a surgical saw adapted for tibial resection. The slot 39 crosses along a cutting plane CP the guide jig 32 so as to enable the surgical saw to cross the guide jig 32. The cutting plane CP is perpendicular to the rotation axis X when the support body 27 is fitted onto the guide jig 32.

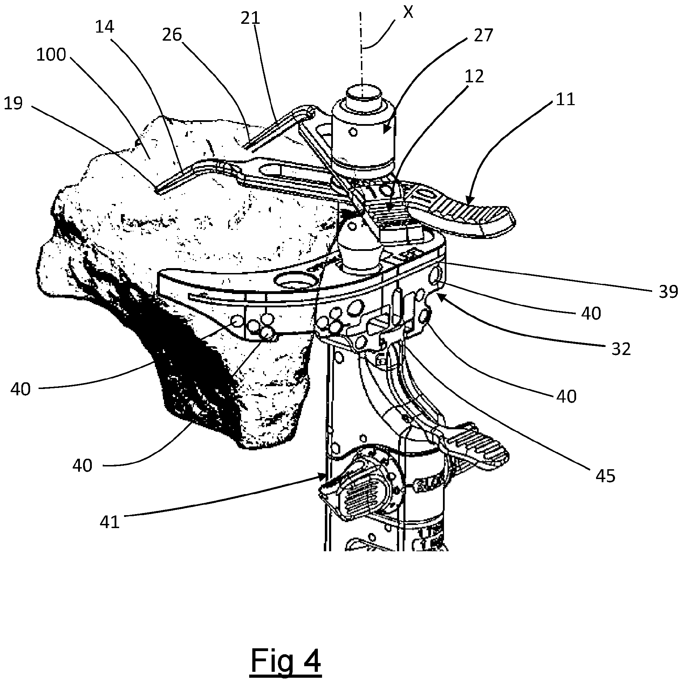

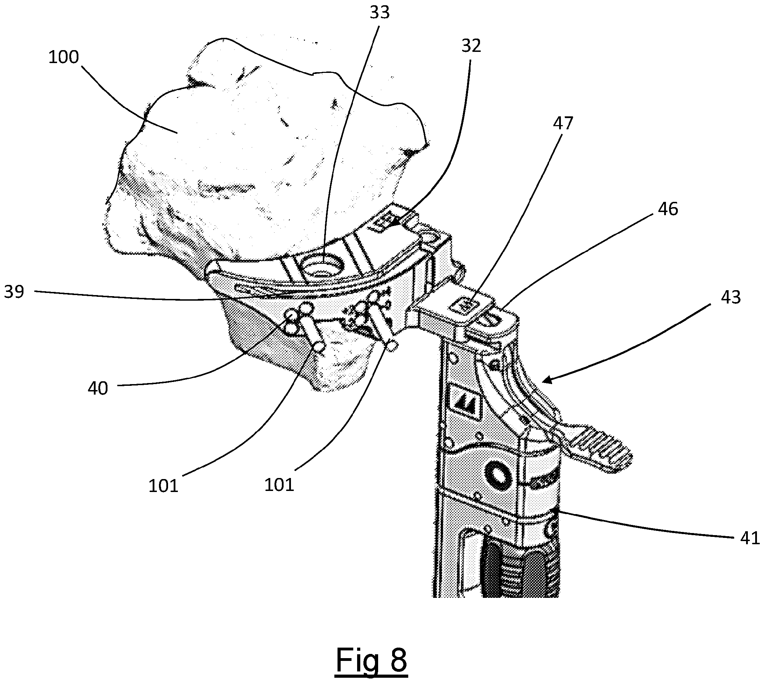

The guide jig 32 is provided with a plurality of through holes 40 (better illustrated in figure 4 and 8) which cross the guide jig 32 along a substantially perpendicular direction to the rotation axis X. In other words, the through holes 40 extend substantially parallel to the cutting plane CP. The through holes 40 are configured to accept anchoring nails 101 (depicted in figure 8) to constrain the guide jig 32 to the patient's tibia, as will be better illustrated below.

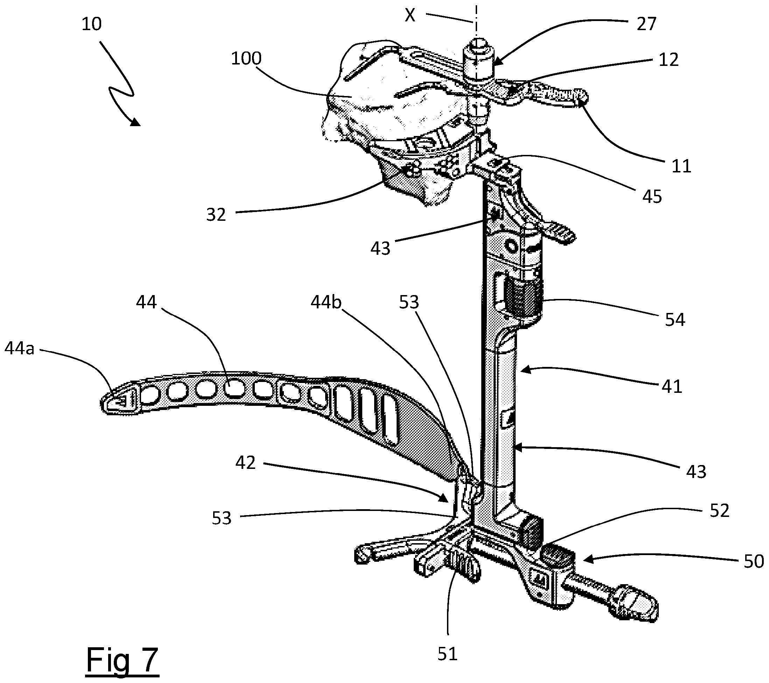

In the embodiment illustrated in the appended drawings, the guide device 10 comprises an extramedullary alignment guide 41 for positioning the guide jig 32 with respect to the patient's tibia. The alignment guide 41 comprises an anchoring portion 42 placed in a distal position and predisposed to be constrained to the patient's ankle and a main portion 43 predisposed to be constrained to the guide jig 32 (figure 7). The extension in the proximal-distal direction of the alignment guide 41 is substantially equal to the length of the patient's tibia.

The anchoring portion 42 comprises a flexible element 44 having two ends 44a, 44b, removably constrained to the alignment guide 41 at the anchoring portion 42. The flexible element 44 is predisposed to be fastened around the patient's ankle, closing in a ring configuration.

The main portion 43 comprises coupling members 45 placed in a proximal position and predisposed to cooperate with the guide jig 32 to constrain the latter to the alignment guide 41 and lock any relative movement between the alignment guide 41 and the guide jig 32. The coupling members 45 are placed on the opposite side of the alignment guide 41 with respect to the anchoring portion 42.

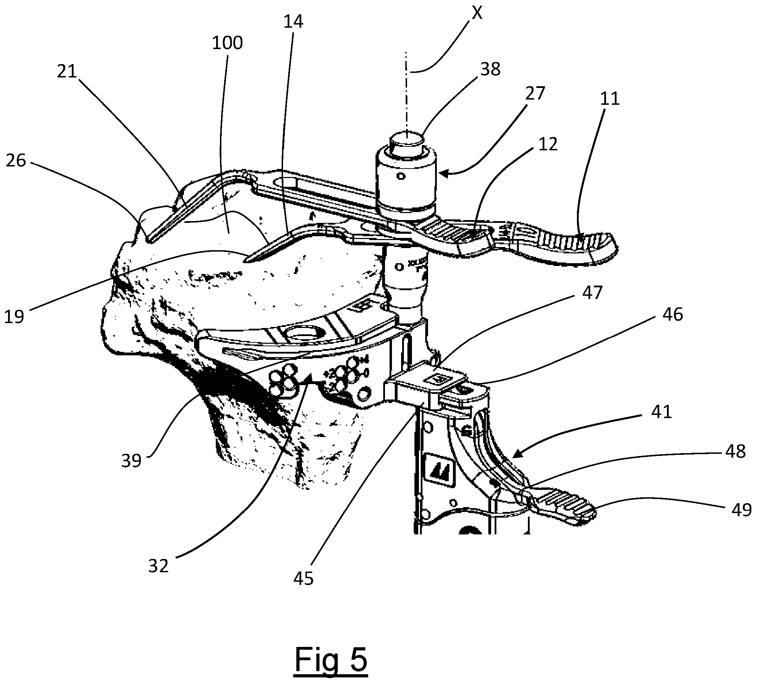

As better illustrated in figure 5, the coupling members 45 comprise a slide 46 which is integral with the alignment guide 41 insertable in a groove 47 provided on the guide jig 32. The insertion of the slide 46 in the groove 47 locks the relative movement between the guide jig 32 and the alignment guide 41 in the medial-lateral direction and in the proximal-distal direction. The coupling members 45 are further provided with a locking and release device 48 that can be switched between a locking condition, in which it prevents the movement of the guide jig 32 with respect to the alignment guide 41 in the anteroposterior direction and therefore prevents the removal of the guide jig 32 from the alignment guide 41, and a release condition in which it enables the sliding in the anteroposterior direction of the guide jig 32 with respect to the alignment guide 41 and therefore the removal of the guide jig 32 from the alignment guide 41.

In the embodiment of figure 5, the locking and release device 48 comprises a movable slider 49 that can be actuated by the surgeon and enables, as a function of the position assumed, the slide 46 to be locked by mechanical interference inside the groove 47 and the sliding of the slide 46 inside the groove 47 (allowing the removal of the slide 46 from the groove 47).

The alignment guide 41 further comprises adjustment members 50 (indicated in figure 7) that enable the relative position of the guide jig 32 to be varied with respect to the patient's tibia.

In particular, the adjustment members 50 enable the guide jig 32 to be translated in the proximal-distal direction, the guide jig to be rotated about an anteroposterior direction and the guide jig 32 to be rotated about a medial-lateral direction.

For that purpose, the adjustment members 50 act between the anchoring portion 42 and the main portion 43 of the alignment guide 41 to vary the mutual position between these two portions. In particular, the adjustment members 50 comprise a first translation mechanism 51 which enables the main portion 43 to translate in the medial-lateral direction with respect to the anchoring portion 42. The first translation mechanism 51 can for example comprise a guide rail (not illustrated) associated with the anchoring portion 42 or the main portion 43 and a sliding groove (not illustrated) for the guide rail associated with the main portion 43 or with the anchoring portion 42, so that the sliding of the guide rail within the sliding groove determines a translation in the medial-lateral direction of the main portion 43 with respect to the anchoring portion 42. The first translation mechanism 51 can also comprise a locking pin (not illustrated) for locking the guide rail with respect to the sliding groove.

Preventing a translation of the guide jig 32 in the medial-lateral direction, for example by retaining it with a hand or fixing the cutting jig 32 to the tibia with a fixing nail, the translation in the medial-lateral direction of the main portion 43 with respect to the anchoring portion 42 causes a rotation of the guide jig 32 about an anteroposterior direction. In fact, it is to be noted that the anchoring portion 42 of the alignment guide 41 cannot translate (being constrained to the patient's ankle) but can rotate (given the constraint actuated by the flexible element 44) and therefore the relative translation between the main portion 43 and the anchoring portion 42 (when the guide jig 32 cannot translate) causes a rotation of the alignment guide 41 and therefore of the guide jig 32.

The adjustment members 50 further comprise a second translation mechanism 52 which enables the main portion 43 to translate in the anteroposterior direction with respect to the anchoring portion 42. In particular, the second translation mechanism 52 enables the main portion 43 to translate in the anteroposterior direction with respect to the first translation mechanism 51 (which cannot perform movements in the anteroposterior direction with respect to the anchoring portion 42). The second translation mechanism 52 can comprise a guide rail (not illustrated) associated with the first translation mechanism 51 or with the main portion 43 of the main portion of the alignment guide 41 and a sliding groove (not illustrated) for the guide rail associated with the main portion 43 or with the first translation mechanism 51, so that the sliding of the guide rail within the sliding groove determines a translation in the anteroposterior direction of the main portion 43 with respect to the first translation mechanism 51 and therefore with respect to the anchoring portion 42. The second translation mechanism 52 can also comprise a locking pin (not illustrated) for locking the guide rail with respect to the sliding groove.

Preventing a translation of the guide jig 32 in the anteroposterior direction, for example by retaining it with a hand or fixing the cutting jig 32 to the tibia with a fixing nail, the translation in the anteroposterior direction of the main portion 43 with respect to the anchoring portion 42 causes a rotation of the guide jig 32 about a medial-lateral direction. In fact, it is to be noted, also in this case, that the anchoring portion 42 of the alignment guide 41 cannot translate (being constrained to the patient's ankle) but can rotate (given the constraint actuated by the flexible element 44) and therefore the relative translation between the main portion 43 and the anchoring portion 42 (when the guide jig 32 cannot translate) causes a rotation of the alignment guide 41 and therefore of the guide jig 32.

The adjustment members 50 further comprise a third translation mechanism 53 which enables the main portion 43 to translate in the proximal-distal direction with respect to the anchoring portion 42. In particular, the third translation mechanism 53 enables the main portion 43 to translate in the proximal-distal direction with respect to the first 51 and the second translation mechanism 52 (which cannot perform movements in the proximal-distal direction with respect to the anchoring portion 42). The third translation mechanism 53 can comprise a guide rail (not illustrated) associated with the second translation mechanism 52 or with the main portion 43 and a sliding groove (not illustrated) for the guide rail associated with the main portion 43 or with the second translation mechanism 52, so that the sliding of the guide rail within the sliding groove determines a translation in the proximal-distal direction of the main portion 43 with respect to the second translation mechanism 52 and therefore with respect to the anchoring portion 42. The third translation mechanism 53 can also comprise a locking pin (not illustrated) for locking the guide rail with respect to the sliding groove. The third translation mechanism 53 can further comprise a fine adjustment device 54 (depicted in figure 7) for precisely adjusting the translation in the proximal-distal direction of the main portion 43 with respect to the anchoring portion 42 of the alignment guide 41.

The translation in the proximal-distal direction of the main portion 43 causes a translation in the same direction and orientation as the guide jig 32.

The guide device 10 can be used in a tibial resection operation that requires a kinematic alignment of the guide jig 32 (i.e. of the slot 39 for the insertion of the surgical blade) with the patient's native joint line.

In particular, should the surgeon wish to perform an assisted and controlled kinematic alignment of the guide jig 32 with the patient's varus/valgus angle, a tibial resection method may be as follows.

A first 11 and a second feeler 12 are selected that have first end portions 14, 21 provided with respective first D1 and second contact directions D2 able, when the feelers 11, 12 are mounted on the guide jig 32, to reach the selected points of the tibial plateau.

The length of the first end portions 14, 21 of the two feelers 11, 12 is selected so as to reach the same height (when the feelers are in use) or to compensate for any cartilaginoid and/or bone wear of the tibial plateau.

The two feelers are then mounted on the support body 27 so that the first feeler 11 lies below the second feeler 12. The support body 27 is assembled on the guide jig 32.

The alignment guide 41 is constrained, through the flexible element 44, to the patient's ankle.

The guide jig 32 with the support body and the feelers 11, 12 is brought to an anterior and proximal portion of the patient's tibia. Such portion of tibia has been previously exposed, with conventional operating techniques, to enable the resection.

The guide jig 32 is coupled to the alignment guide 41 and the surgeon positions the first and the second feeler 11, 12 at areas or points of the medial condyle and of the lateral condyle that identify, on a parallel plane to the front plane, the patient's native joint line.

The alignment guide 41 is adjusted by acting on the adjustment members 50 for creating an optical alignment of the guide jig 32 (and in particular of the slot 39) with the slope angle of the patient's tibia.

During this step, the mentioned alignment is not assisted and controlled, i.e. it is performed by looking for and finding an alignment through an optical comparison between the orientation of the slot 39 and an imaginary line that joins two separate points in the anteroposterior direction of the medial condyle and of the lateral condyle that the surgeon considers to be representative of the slope angle.

The alignment of the guide jig 32 with the slope angle is performed by acting on the second translation mechanism 52 with the guide jig 32 which cannot translate in the anteroposterior direction. After obtaining the desired alignment, the surgeon acts on the adjustment members 50 to obtain an assisted and controlled alignment of the guide jig 32 (i.e. of the slot 39) with the native varus/valgus angle and to position the guide jig at a predetermined cutting height.

In particular, the surgeon acts on the first adjustment mechanism 51 and on the third adjustment mechanism 53 (and possibly on the fine adjustment device 54) until the contact areas 19, 26 of the first 11 and the second feeler 12 are in contact with pre-selected areas or points of the medial condyle or the lateral condyle, as schematically illustrated in figure 4.

At this point, in some or all of the through holes 40 in the guide jig 32, anchoring nails 101 are inserted, as illustrated in figure 8 and subsequently the support guide 27 and with them the first and the second feeler 11, 12 are removed from the guide jig 32. The anchoring nails 101 are screwed into the patient's tibia so that the guide jig 32 is perfectly integral with the tibia and cannot move with respect to it.

The alignment guide 41 can be removed and the slot 39 of the guide jig 32 can be engaged by a surgical saw for performing the tibial resection. During the resection, the slot 39 acts as a guide for the inclination of the surgical saw with respect to the tibia. Should the surgeon wish to perform an assisted and controlled kinematic alignment of the guide jig 32 with the patient's slope angle, a tibial resection method may be as follows.

A first 11 and a second feeler 12 are selected that have first end portions 14, 21 provided with respective first D1 and second contact directions D2 able, when the feelers 11, 12 are mounted on the guide jig 32, to reach the selected points of the tibial plateau.

The length of the first end portions 14, 21 of the two feelers 11, 12 is selected so as to reach the same height (when the feelers are in use) or to compensate for any cartilaginoid and/or bone wear of the tibial plateau.

The two feelers 11, 12 are then mounted on the support body 27 so that the first feeler 11 lies below the second feeler 12. The support body 27 is assembled on the guide jig 32.

The alignment guide 41 is constrained, through the flexible element 44, to the patient's ankle.

The guide jig 32 with the support body 27 and the feelers 11, 12 is brought to an anterior and proximal portion of the patient's tibia. Such portion of tibia has been previously exposed, with conventional operating techniques, to enable the resection.

The guide jig 32 is coupled to the alignment guide 41 and the surgeon positions the first and the second feeler 11, 12 at the pre-selected points on the same condyle (medial or lateral) separated in the anteroposterior direction.

The alignment guide 41 is adjusted by acting on the adjustment members 50 for creating an optical alignment of the guide jig 32 (and in particular of the slot 39) with the varus/valgus angle of the patient's tibia.

During this step, the mentioned alignment is not assisted and controlled, i.e. it is performed by looking for and finding an alignment through an optical comparison between the orientation of the slot 39 and an imaginary line that joins two points of the medial condyle and of the lateral condyle that the surgeon considers to be representative of the native varus/valgus angle. The alignment of the guide jig 32 with the varus/valgus angle is performed by acting on the first translation mechanism 51 with the guide jig 32 which cannot translate in the medial-lateral direction.

After obtaining the desired alignment, the surgeon acts on the adjustment members 50 to obtain an assisted and controlled alignment of the guide jig 32 (i.e. of the slot 39) with the slope angle and to position the guide jig at a predetermined cutting height.

In particular, the surgeon acts on the second adjustment mechanism 52 and on the third adjustment mechanism 53 (and possibly on the fine adjustment device 54) until the contact areas 19, 26 of the first 11 and the second feeler 12 are in contact with pre-selected points of the medial condyle or the lateral condyle, as schematically illustrated in figure 5.

At this point, in some or all of the through holes 40 in the guide jig 32, anchoring nails 101 are inserted, as illustrated in figure 8 and subsequently the support guide 27 and with them the first and the second feeler 11, 12 are removed from the guide jig 32. The anchoring nails 101 are screwed into the patient's tibia so that the guide jig 32 is perfectly integral with the tibia and cannot move with respect to it.

The alignment guide 41 can be removed and the slot 39 of the guide jig 32 can be engaged by a surgical saw for performing the tibial resection. During the resection, the slot 39 acts as a guide for the inclination of the surgical saw with respect to the tibia. Obviously, a person skilled in the art, for the purpose of fulfilling specific and contingent needs, can make numerous modifications and variations to the invention described above, such as, for example, to have the guide device be operated by an authorised user instead of a surgeon, all however contained within the scope of protection of the present invention as defined by the following claims.

## Claims

1. Tibial resection guide device (10) comprising:
a guide jig (32) comprising a slot (39) provided for accepting and guiding a surgical saw;
a first feeler (11) provided for coming into contact with a point or an area of a tibial plate (100);
a second feeler (12) provided for coming into contact with a point or an area of a tibial plate (100);
wherein the first feeler (11) and the second feeler (12) can be rotated with respect to one another and to the guide jig (32) around the same rotation axis (X), wherein the first feeler (11) can be translated with respect to the second feeler (12) and to the guide jig (32) along a first sliding direction (S1) contained within a first sliding plane (P1), and wherein the second feeler (12) can be translated with respect to the first feeler (11) and to the guide jig (32) along a second sliding direction (S2) contained within a second sliding plane (P2) parallel to the first sliding plane (P1).

2. Guide device (10) according to claim 1, wherein said rotation axis (X), the first sliding plane (P1) and the second sliding plane (P2) have respective pre-set inclinations relative to the slot (39) of the guide jig (32).

3. Guide device (10) according to claim 1 or 2, wherein the first sliding plane (P1) and the second sliding plane (P2) are perpendicular to said rotation axis (X).

4. Guide device (10) according to any one of the previous claims, wherein the first feeler (11) comprises a first end portion (14) that develops along a first contact direction (D1), inclined with respect to the first sliding plane (P1), the first end portion (14) being configured to come into contact with a point or an area of a tibial plate (100), the projection of the first contact direction (D1) on the first sliding plane (P1) being inclined with respect to the first sliding direction (S1).

5. Guide device (10) according to any one of the previous claims, wherein the second feeler (12) comprises a first end portion (21) that develops along a second contact direction (D2) inclined with respect to the second sliding plane (P2), the first end portion (21) being configured to come into contact with a point or an area of a tibial plate (100), the projection of the second contact direction (D2) on the second sliding plane (P2) being inclined with respect to the second sliding direction (S2).

6. Guide device (10) according to claims 4 and 5, wherein the first feeler (11) is spaced apart by a pre-set distance along the rotation axis (X) from the second feeler (12), the first end portion (14) of the first feeler (11) having an extension, in a direction parallel to the rotation axis (X), that is equivalent to the extension, in a direction parallel to the rotation axis (X), of the first end portion (21) of the second feeler (12) reduced by a pre-set amount.

7. Guide device (10) according to any one of the previous claims, comprising a support body (27) for the first (11) and the second feeler (12) that can be inserted into the guide jig (32), the support body (27) comprising tightening members (31) that can be switched from one free positioning condition, in which the first (11) and the second feeler (12) can be rotated and translated with respect to the support body (27), and a locking condition, in which the first (11) and the second (12) feeler are held in position with respect to the support body (27).

8. Guide device (10) according to claim 7, wherein said guide jig (32) comprises a plurality of constraining members (33) spaced apart from one another to accept and hold the support body (27) in different positions.

9. Guide device (10) according to any one of the previous claims comprising an extramedullary or intramedullary alignment guide (41), said guide jig (32) being associated with said alignment guide (41) so that it can be moved by said alignment guide (41).

10. Guide device (10) according to claim 9, wherein the alignment guide (41) comprises adjustment members (50) to translate the guide jig (32) along a direction parallel to said rotation axis (X) and to rotate the guide jig (32) around two axes that are perpendicular to one another and perpendicular to the rotation axis (X).

## Patentansprüche

1. Tibiaresektionsführung (10), umfassend:
eine Halteführung (32), umfassend einen Schlitz (39), der zur Aufnahme und Führung einer chirurgischen Säge vorgesehen ist;
einen ersten Taster (11), der dazu vorgesehen ist, mit einem Punkt oder einem Bereich einer Tibiaplatte (100) in Kontakt zu kommen;
einen zweiten Taster (12), der dazu vorgesehen ist, mit einem Punkt oder einem Bereich einer Tibiaplatte (100) in Kontakt zu kommen;
wobei der erste Taster (11) und der zweite Taster (12) in Bezug aufeinander und auf die Halteführung (32) um dieselbe Drehachse (X) gedreht werden können, wobei der erste Taster (11) in Bezug auf den zweiten Taster (12) und auf die Halteführung (32) entlang einer ersten Gleitrichtung (S1), die in einer ersten Gleitebene (P1) enthalten ist, verschoben werden kann und
wobei der zweite Taster (12) in Bezug auf den ersten Taster (11) und auf die Halteführung (32) entlang einer zweiten Gleitrichtung (S2), die in einer zweiten Gleitebene (P2) parallel zur ersten Gleitebene (P1) enthalten ist, verschoben werden kann.

2. Führung (10) nach Anspruch 1, wobei die Drehachse (X), die erste Gleitebene (P1) und die zweite Gleitebene (P2) jeweils vorgegebene Neigungen relativ zum Schlitz (39) der Halteführung (32) aufweisen.

3. Führung (10) nach Anspruch 1 oder 2, wobei die erste Gleitebene (P1) und die zweite Gleitebene (P2) senkrecht zur Drehachse (X) stehen.

4. Führung (10) nach einem der vorstehenden Ansprüche, wobei der erste Taster (11) einen ersten Endabschnitt (14) umfasst, der sich entlang einer ersten Kontaktrichtung (D1) erstreckt, die in Bezug auf die erste Gleitebene (P1) geneigt ist, wobei der erste Endabschnitt (14) konfiguriert ist, um mit einem Punkt oder einem Bereich einer Tibiaplatte (100) in Kontakt zu kommen, wobei die Projektion der ersten Kontaktrichtung (D1) auf die erste Gleitebene (P1) in Bezug auf die erste Gleitrichtung (S1) geneigt ist.

5. Führung (10) nach einem der vorstehenden Ansprüche, wobei der zweite Taster (12) einen ersten Endabschnitt (21) umfasst, der sich entlang einer zweiten Kontaktrichtung (D2) entwickelt, die gegenüber der zweiten Gleitebene (P2) geneigt ist, wobei der erste Endabschnitt (21) konfiguriert ist, um mit einem Punkt oder einem Bereich einer Tibiaplatte (100) in Kontakt zu kommen, wobei die Projektion der zweiten Kontaktrichtung (D2) auf die zweite Gleitebene (P2) in Bezug auf die zweite Gleitrichtung (S2) geneigt ist.

6. Führung (10) nach einem der Ansprüche 4 und 5, wobei der erste Taster (11) um einen vorgegebenen Abstand entlang der Drehachse (X) von dem zweiten Taster (12) beabstandet ist, wobei der erste Endabschnitt (14) des ersten Tasters (11) eine Erstreckung in einer Richtung parallel zur Drehachse (X) aufweist, die in einer Richtung parallel zur Drehachse (X) äquivalent zu der Erstreckung des ersten Endabschnitts (21) des zweiten Tasters (12) ist, der um ein vorgegebenes Maß verringert ist.

7. Führung (10) nach einem der vorstehenden Ansprüche, umfassend einen Trägerkörper (27) für den ersten (11) und den zweiten Taster (12), der in die Führung (32) eingesetzt werden kann, wobei der Trägerkörper (27) Spannelemente (31) umfasst, die zwischen einem freien Positionierungszustand, in dem der erste (11) und der zweite Taster (12) in Bezug auf den Trägerkörper (27) gedreht und verschoben werden können, und einem Verriegelungszustand, in dem der erste (11) und der zweite (12) Taster in Bezug auf den Trägerkörper (27) in Position gehalten werden, geschaltet werden können.

8. Führung (10) nach Anspruch 7, wobei die Halteführung (32) eine Vielzahl von Zwangselementen (33) umfasst, die voneinander beabstandet sind, um den Trägerkörper (27) in verschiedenen Positionen aufzunehmen und zu halten.

9. Führung (10) nach einem der vorstehenden Ansprüche mit einer extramedullären oder intramedullären Ausrichtungsführung (41), wobei die Halteführung (32) mit der Ausrichtungsführung (41) verbunden ist, so dass sie durch die Ausrichtungsführung (41) bewegt werden kann.

10. Führung (10) nach Anspruch 9, wobei die Ausrichtungsführung (41) Einstellelemente (50) umfasst, um die Halteführung (32) entlang einer Richtung parallel zu der Drehachse (X) zu verschieben und die Halteführung (32) um zwei Achsen zu drehen, die senkrecht zueinander und senkrecht zu der Drehachse (X) sind.

## Revendications

1. Dispositif de guidage pour une résection tibiale (10) comprenant :
un gabarit de guidage (32) comprenant une fente (39) prévue pour accepter et guider une scie chirurgicale ;
un premier palpeur (11) prévu pour venir en contact avec un point ou une zone d'une plaque tibiale (100) ;
un second palpeur (12) prévu pour venir en contact avec un point ou une zone d'une plaque tibiale (100) ;
dans lequel le premier palpeur (11) et le second palpeur (12) peuvent tourner l'un par rapport à l'autre et par rapport au gabarit de guidage (32) autour du même axe de rotation (X), dans lequel le premier palpeur (11) peut être déplacé par rapport au second palpeur (12) et au gabarit de guidage (32) le long d'une première direction de glissement (S1) contenue dans un premier plan de glissement (P1), et dans lequel le deuxième palpeur (12) peut être déplacé par rapport au premier palpeur (11) et au gabarit de guidage (32) le long d'une deuxième direction de glissement (S2) contenue dans un deuxième plan de glissement (P2) parallèle au premier plan de glissement (P1).

2. Dispositif de guidage (10) selon la revendication 1, dans lequel ledit axe de rotation (X), le premier plan de glissement (P1) et le second plan de glissement (P2) ont des inclinaisons respectives prédéfinies par rapport à la fente (39) du gabarit de guidage (32).

3. Dispositif de guidage (10) selon la revendication 1 ou 2, dans lequel le premier plan de glissement (P1) et le second plan de glissement (P2) sont perpendiculaires au dit axe de rotation (X).

4. Dispositif de guidage (10) selon l'une quelconque des revendications précédentes, dans lequel le premier palpeur (11) comprend une première portion d'extrémité (14) qui se développe selon une première direction de contact (D1), inclinée par rapport au premier plan de glissement (P1), la première portion d'extrémité (14) étant configurée pour venir en contact avec un point ou une zone d'une plaque tibiale (100), la projection de la première direction de contact (D1) sur le premier plan de glissement (P1) étant inclinée par rapport à la première direction de glissement (S1).

5. Dispositif de guidage (10) selon l'une quelconque des revendications précédentes, dans lequel le deuxième palpeur (12) comprend une première portion d'extrémité (21) qui se développe selon une deuxième direction de contact (D2), inclinée par rapport au deuxième plan de glissement (P2), la première portion d'extrémité (21) étant configurée pour venir en contact avec un point ou une zone d'une plaque tibiale (100), la projection de la deuxième direction de contact (D2) sur le deuxième plan de glissement (P2) étant inclinée par rapport à la deuxième direction de glissement (S2).

6. Dispositif de guidage (10) selon les revendications 4 et 5, dans lequel le premier palpeur (11) est espacé d'une distance prédéfinie selon l'axe de rotation (X) du deuxième palpeur (12), la première partie d'extrémité (14) du premier palpeur (11) ayant une extension, selon une direction parallèle à l'axe de rotation (X), équivalente à l'extension, selon une direction parallèle à l'axe de rotation (X), de la première partie d'extrémité (21) du deuxième palpeur (12), réduite d'une quantité prédéfinie.

7. Dispositif de guidage (10) selon l'une quelconque des revendications précédentes, comprenant un corps de support (27) pour le premier (11) et le second palpeur (12), qui peut être inséré dans le gabarit de guidage (32), le corps de support (27) comprenant des éléments de serrage (31) qui peuvent être commutés d'un état de positionnement libre, dans lequel le premier (11) et le second palpeur (12) peuvent être tournés et déplacés par rapport au corps de support (27), et un état de verrouillage, dans lequel le premier (11) et le second (12) palpeur sont maintenus en position par rapport au corps de support (27).

8. Dispositif de guidage (10) selon la revendication 7, dans lequel ledit gabarit de guidage (32) comprend une pluralité d'éléments de contrainte (33) espacés les uns des autres pour accepter et maintenir le corps de support (27) dans différentes positions.

9. Dispositif de guidage (10) selon l'une quelconque des revendications précédentes comprenant un guide d'alignement extramédullaire ou intramédullaire (41), ledit gabarit de guidage (32) étant associé au dit guide d'alignement (41) de manière à pouvoir être déplacé par ledit guide d'alignement (41).

10. Dispositif de guidage (10) selon la revendication 9, dans lequel le guide d'alignement (41) comprend des éléments de réglage (50) pour translater le gabarit de guidage (32) le long d'une direction parallèle au dit axe de rotation (X) et pour faire tourner le gabarit de guidage (32) autour de deux axes qui sont perpendiculaires l'un à l'autre et perpendiculaires à l'axe de rotation (X).
